# EUROPEAN PATENT SPECIFICATION

(11) **EP 3 032 254 B1**
(45) Date of publication and mention of the grant of the patent: **14.12.2016**
(21) Application number: 15198455.6
(22) Date of filing: 08.12.2015
(51) Int. Cl.: G01N 33/00, G01N 1/22, G01N 1/40

(54) **METHOD AND DEVICE FOR DETECTION OF ELEMENTAL GASEOUS MERCURY IN AIR OR IN OTHER GASES**
VERFAHREN UND VORRICHTUNG ZUR ERKENNUNG GASFÖRMIGEN QUECKSILBERS IN DER LUFT ODER IN ANDEREN GASEN
PROCÉDÉ ET DISPOSITIF POUR LA DÉTECTION DE MERCURE ÉLÉMENTAIRE GAZEUX DANS L'AIR OU DANS D'AUTRES GAZ

(30) Priority: 12.12.2014 LT 2014513
(43) Date of publication of application: 15.06.2016
(73) Proprietor: Valstybinis moksliniu tyrimu institutas Fiziniu ir technologijos mokslu centras, 02300 Vilnius (LT)
(72) Inventor: Urba, Andriejus, 09111 Vilnius (LT); Kvietkus, Kestutis, 08308 Vilnius (LT); Sakalys, Jonas, 02309 Vilnius (LT); Didzbalis, Jonas, 08406 Vilnius (LT); Valiulis, Darius, 10218 Vilnius (LT)
(74) Representative: Draugeliene, Virgina Adolfina

(56) References cited:
- US-A1- 2013 236 361
- OIVA I. JOENSUU: "Mercury-Vapor Detector", APPLIED SPECTROSCOPY, vol. 25, no. 5, 1971, pages 526-528, XP055184989, ISSN: 0003-7028, DOI: 10.1366/000370271779950481

## Description

### Technical field

The present invention relates to the field of material analysis and air pollution research, more specifically to a method and device for determination of elemental mercury concentration in the air or in other gases.

### Background Art

It is known an apparatus for detection of mercury vapor in gases, comprising a device for accumulating of mercury which includes a quartz tube with a gold trap inside the tube made of the gold wire and supplied with a heating coil externally. Described in the Lithuanian patent LT 3138 B, 09-02-1993.

The nearest known method according to the technical field is a method comprising a sequence of operations described below. A predetermined volume of a sample air or other gases is passed through a collection zone of a quartz tube, said collection zone is formed of the gold structure. During the passing period, collecting of mercury particles from the sample gas takes place. Within the period of collecting temperature of the collection zone is maintained above ambient temperature but below 100°C. The collected elemental gaseous mercury then is desorbed by heating the collection zone of the quartz tube together with the gold structure inside, up to the desorption temperature which is in the range 500°C to 600°C. The desorbed mercury vapor together with possibly desorbed other gaseous admixtures are passed with a stream of carrier gas to an optic detection zone of a mercury analyzer (Described in US patent No. US5597535, 25-02-1994, TEKRAN INC).

It is known an apparatus for the detection of elemental mercury vapor in air or in other gases, which comprises a quartz tube inside of which is placed a collection zone formed of gold structure, designed to collect and then desorb mercury vapor from a sample of air or other gases. The heating means is provided to keep a predetermined temperatures respectively for the collecting step and for the desorption step. Further the means for passing the desorbate to the optical detector unit with a carrier gas stream is provided. (See patent US5597535, 1994-02-25, TEKRAN INC).

Disadvantage of the above described method and apparatus is that besides mercury vapor also other gaseous admixtures can be contained in the air sample in particular, the VOCs, (Volatile Organic Compounds), which can be partly adsorbed on the surface of gold structure and later desorbed and/or pyrolyzed by heating to Hg desorption temperature in the inert carrier gas atmosphere, may pollute the gold surface and also the optical detector and cause interference to measurements.

It is further known from the article Oiva I. Joensuu, "Mercury-Vapor Detector", Appl Spectrosc September 1971 25: 526-528, doi:10.1366/000370271779950481, to destroy interfering organic compounds by oxidation.

### Technical problem to be solved

The present invention seeks to improve the precision of gaseous elemental mercury detection by avoiding pollution of the system with VOCs and abating interference to measurements by VOCs.

### Disclosure of invention

According to the present invention there is proposed a method for detection of elemental gaseous mercury in air or in other gases, comprising following steps: collection of elemental gaseous mercury by passing a predetermined volume of a sample of air or other gases through a collection zone formed inside a quartz tube, where said collection zone is formed as a gold structure; maintaining of a predetermined temperature of the collection zone during the collection step; desorption of the collected elemental gaseous mercury by heating the collection zone of the quartz tube up to desorption temperature and transmitting of the desorbed elemental gaseous mercury by a carrier gas stream to a mercury analyzer, wherein the desorbed elemental gaseous mercury together with other possibly desorbed gaseous admixtures in the air or other gases are passed through a zone of oxidation, which is heated up and maintained at temperature in a range from 350°C to 1000°C, preferably in a range from 900°C to 1000°C, and more preferably at 960°C during the desorption and oxidation step, whereas temperature of oxidation zone is maintained in a range from ambient temperature to 500°C during the collection step, and the carier gas, transmitting the desorbed elemental gaseous mercury together with other possibly desorbed gaseous admixtures from the collection zone through the oxidation zone to the mercury analyzer, has an oxygen content not less as in air. The collection zone of the quartz tube during collection step may be maintained at temperature in a range from 100°C to 220°C, preferably at 200°C.

According to another embodiment of the present invention there is provided a device for detection of gaseous elemental mercury, comprising: a quartz tube, inside of which a collection zone is formed from a gold structure for collecting and subsequent desorbing the gaseous elemental mercury from a sample of air or other gases, which passes through the quartz tube; a heating means to maintain predetermined temperature of the collection zone during the collection and to heat up and to to maintain predetermined temperature during the desorption; a means for passing of the desorbed mercury vapor to the mercury analyzer by a carrier gas stream, wherein an oxidation zone is formed downstream from the collection zone inside the quartz tube, which is provided with a separate heating means for heat up and to maintain predetermined temperature of the oxidation zone in a range from 350°C to 1000°C, preferably in a range from 900°C to 1000°C, more preferably at 960°C during the desorption and oxidation steps, and in the range from 100°C to 500°C during the collection step.

The oxidation zone inside the quartz tube may be formed from a parallel or other quartz structure. The oxidation zone may be an empty part of predetermined lenght of the quartz tube downstream of the collection zone. The oxidation zone may be formed with one or several catalytic compounds, such as base metal oxides, titanium dioxide, metals of platinum group or other catalytic compounds, whereas temperature of the oxidation zone during the desorption and oxidation steps is maintained in the range from 350°C to 900°C.

According to the present invention in proposed method and device the desorbed gaseous admixtures, in particular VOCs completely burn up and the products of reaction (generally, H₂O and CO₂) would not pollute the downstream tubes and would not effect the optic atomic absorption detector at the 253,7 nm UV Hg spectral feature or their influence would be minimal. Seeking to avoid undesirable adsorption of gaseous admixtures within the oxidation zone during the collection step, the said oxidation zone is also maintained at the temperature in the range from 100°C to 500°C also during the collection step. The collection zone is maintained at 200°C during the collection step which minimizes unselective and undesirable adsorption of other gaseous atmixtures, in particular the VOCs, still allowing to adsorb the elemental gaseous mercury. All these measures help to abate access of the VOCs to the optic detector and thus improve precision of gaseous elemental mercury detection.

### Brief description of drawings

The invention is explained by the drawings in which the given illustrations are possible but not limiting realizations of the present invention.
Fig.1 - is a schematic view of the quartz tube inside of which is formed the collection zone made of gold structure and oxidation zone formed as empty part of the quartz tube of the proposed device.
Fig.2 - is a schematic view of the quartz tube inside of which is formed the collection zone made of gold structure and oxidation zone made of parallel or other quartz structure or one or several catalytic compounds of the proposed device

Detailed description of at least one way of carrying out of the invention.

According to the present invention a method for the detection of elemental gaseous mercury in air or in other gases comprises a sequence of steps described below. A predetermined volume of sample air or other gases is passed through a collection zone formed inside of a quartz tube, where the collection zone is formed as a gold structure and which is maintained at the temperature of about 200°C to minimize non selective adsorption of different gaseous admixtures such as VOCs still allowing to adsorb mercury. During the collection step, the temperature of the oxidation zone is maintained in the range from about 100°C to 500°C, depending also from the design of oxidation zone (quartz structure, or empty, or catalytic) used inside. Next step is mercury desorption which is achieved by heating the collection zone up to the desorption temperature in the range 500°C to 600°C. Prior to starting desorption of the collection zone, the oxidation zone is heated up and maintained at about 960°C if it is empty quartz tube, or this temperature can be somewhat lower if the oxidizing zone is made with catalytic compound(s). The desorbed gaseous mercury together with some other possible gaseous admixtures are passing the heated oxidation zone by a carrier gas. The carrier gas contains oxygen not lower amount than the air. The different gaseous admixtures are burned up in the oxidation zone and the gaseous mercury reaches the optic detection zone of the mercury analyzer.

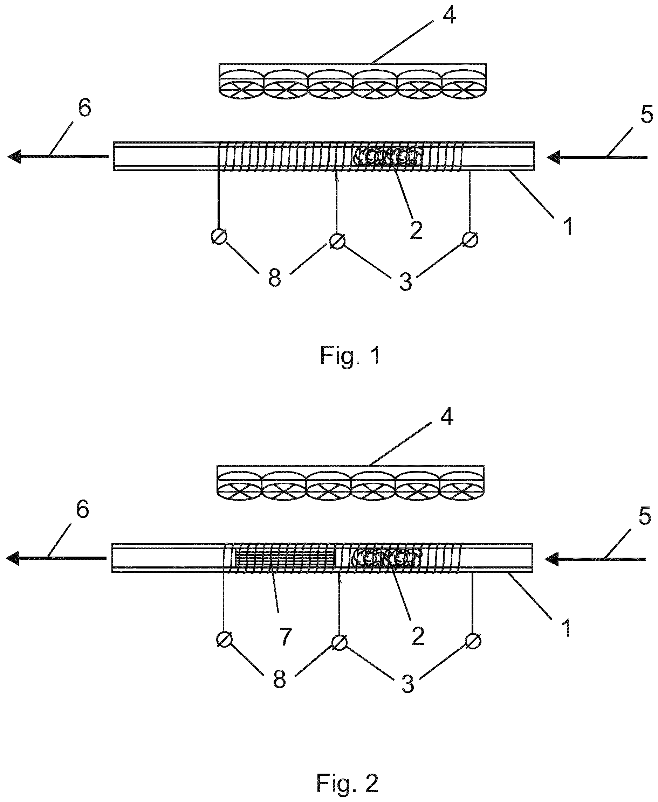

According to the present invention a device for the detection of elemental gaseous mercury in air or in other gases comprises a quartz tube 1 inside of which a collection zone is formed from the gold structure 2, and externally the collection zone is provided with the heating means 3. Fans 4 are intended to cool down the quartz tube 1 after each heating cycle. The sampled air or other sampled gas and also carrier gas enter the quartz tube 1 at its inlet 5. Downstream of the collection zone 2 the oxidation zone is formed and supplied with the coil for heating 8. In Fig.1 the oxidation zone is formed as a empty part of predetermined length of the quartz tube 1. In Fig.2 the oxidation zone is made of the parallel or other quartz or catalytic compound structure 7. The carrier gas stream from the quartz tube 1 outlet 6 is fed to the optic detection zone of the mercury analyzer (not shown in figures).

Principle of operation of the device. During the collection step, certain period of time, predetermined volume of sample of air or other gases 5 are passed through the quartz tube 1 through its inlet. Mercury is collected, by maintaining the predefined temperatures of collection and oxidation zones with the coils 3 and 8 respectively. Prior to starting the desorption step, the temperature of the oxidation zone is increased and maintained during the desorption and oxidation steps about 900°C by heating means 8. Then the collection zone 2 is heated up by the coil 3 to the desorption temperature in the range from 500°C to 600°C and is maintained during the desorption step. The desorbed gaseous mercury together with also possibly desorbed some other gaseous admixtures, such as VOCs, pass with the containing oxygen carrier gas stream the oxidation zone, where the admixtures burn up, at which time mercury vapor passes unaffected and is fed to the optic detection zone of the mercury analyzer (not shown in figure).

## Claims

1. Method for detection of elemental gaseous mercury in air or in other gases, comprising following steps:
- collection of elemental gaseous mercury by passing a predetermined volume of a sample of air or other gases through a collection zone formed inside a quartz tube, where said collection zone is formed as a gold structure,
- maintaining of a predetermined temperature of the collection zone during the collection step,
- desorption of the collected elemental gaseous mercury by heating the collection zone of the quartz tube up to desorption temperature,
- transmitting of the desorbed elemental gaseous mercury by a carrier gas stream to a mercury analyzer,
- passing the desorbed elemental gaseous mercury together with other possibly desorbed gaseous admixtures in the air or other gases before transmitting to a mercury analizer through a zone of oxidation, **characterized in that** the zone of oxidation is heated up before the desorption step and maintained at temperature in a range from 350°C to 1000°C, preferably in a range from 900°C to 1000°C, and more preferably at 960°C during the desorption and oxidation step, whereas the temperature of oxidation zone is maintained in a range from ambient temperature to 500°C during the collection step, and the carrier gas, transmitting the desorbed elemental gaseous mercury together with other possibly desorbed gaseous admixtures from the collection zone through the oxidation zone to the mercury analyzer, has an oxygen content not less as in air.

2. Method according to claim 1, **characterized in that** the collection zone of the quartz tube during collection step is maintained at a temperature within a range from 100°C to 220°C, preferably at 200°C.

3. Device for detection of gaseous elemental mercury in air or in other gases, comprising:
- a quartz tube 1, inside of which a collection zone is formed from a gold structure 2 for collecting and subsequent desorbing the gaseous elemental mercury from a sample of air or other gases, which passes through the quartz tube 1,
- a heating means 3 to maintain predetermined temperature of the collection zone during the collection and to heat up and to maintain predetermined temperature during the desorption,
- a means for passing of the desorbed gaseous elemental mercury to the mercury analyzer by a carrier gas stream, **characterized in that** an oxidation zone is formed downstream from the collection zone inside the quartz tube 1, which is provided with a separate heating means 8 for heating up and to maintaining a predetermined temperature of the oxidation zone in a range of 350°C to 1000°C, preferably in a range from 900°C to 1000°C, more preferably at 960°C during the desorption and oxidation step, and in the range from 100°C to 500°C during the collection step.

4. Device according to claim 3, **characterized in that** the oxidation zone inside the quartz tube is formed from a parallel or other quartz structure.

5. Device according to claim 3, **characterized in that** the oxidation zone is an empty part of predetermined lenght of the quartz tube downstream of the collection zone.

6. Device according to claim 3, **characterized in that** the oxidation zone is formed with one or several catalytic compounds, such as base metal oxides, titanium dioxide, metals of platinum group or other catalytic compounds, whereas temperature of the oxidation zone during the desorption and oxidation steps is maintained in the range from 350°C to 900°C.

## Patentansprüche

1. Verfahren zum Nachweis von elementarem gasförmigem Quecksilber in Luft oder in anderen Gasen, mit folgenden Schritten:
- Sammeln von elementarem gasförmigem Quecksilber durch Durchleiten eines vorbestimmten Volumens einer Probe von Luft oder anderen Gasen durch eine Sammelzone, die in einem Quarzrohr gebildet ist, wobei die Sammelzone als eine Goldstruktur ausgebildet ist,
- Aufrechterhalten einer vorbestimmten Temperatur der Sammelzone während des Sammelschritts,
- Desorption des gesammelten elementaren gasförmigen Quecksilbers durch Erhitzung der Sammelzone des Quarzrohrs bis zu Desorptionstemperatur,
- Übertragen des desorbierten elementaren gasförmigen Quecksilbers durch einen Trägergasstrom zu einem Quecksilberanalysator,
- Weitergeben des desorbierten elementaren gasförmigen Quecksilbers zusammen mit anderen möglicherweise desorbierten gasförmigen Beimengungen in der Luft oder anderen Gasen durch eine Oxidationszone vor dem Übertragen zu einem Quecksilberanalysator
**dadurch gekennzeichnet, dass**
die Oxidationszone vor dem Desorptionsschritt erwärmt und bei der Temperatur im Bereich von 350 ° C bis 1000 ° C, vorzugsweise im Bereich von 900 ° C bis 1000 ° C und besonders bevorzugt bei 960 ° C während des Desorptions- und Oxidationsschrittes gehalten wird,
die Temperatur der Oxidationszone in einem Bereich von Umgebungstemperatur bis 500 ° C während des Sammelschritts,
und das Trägergas, das desorbierte elementare gasförmige Quecksilber zusammen mit anderen ggf. desorbierten Gasmischungen von der Sammelzone durch die Oxidationszone zum Quecksilber-Analysator überleitet, einen Sauerstoffgehalt nicht weniger als in der Luft hat.

2. Verfahren nach Anspruch 1, **dadurch gekennzeichnet, dass** während des Sammelschritts die Temperatur in der Sammelzone des Quarzrohrs im Bereich zwischen 100°C und 220°C, vorzugsweise bei 200°C, liegt.

3. Vorrichtung zum Nachweis von gasförmigem elementarem Quecksilber in Luft oder in anderen Gasen, umfassend:
- ein Quarzrohr 1, innerhalb dessen eine Sammelzone aus einer Goldstruktur 2 zum Sammeln und anschließenden Desorbieren des gasförmigen elementaren Quecksilbers aus einer Probe von Luft oder anderen durch das Quarzrohr 1 hindurchgehen Gasen gebildet ist,
- eine Heizmittel 3 zum Aufrechterhalten einer vorbestimmten Temperatur in der Sammelzone während des Sammelns und zum Aufheizen und zum Aufrechterhalten einer vorbestimmten Temperatur während der Desorption,
- ein Mittel zum Durchleiten des desorbierten gasförmigen elementaren Quecksilbers zu dem Quecksilberanalysator durch einen Trägergasstrom, **dadurch gekennzeichnet,**
**dass** die Oxidationszone stromab von der Sammelzone im Quarzrohr (1) ausgebildet ist, während das Quarzrohr ausgestattet wird mit eigenen Heizmitteln (8) zur Erhitzung und zum Aufrechterhalten der vorbestimmten Temperatur in der Oxidationszone im Bereich von 350°C bis 1000°C, vorzugsweise von 900°C bis 1000°C, bestens bei 960 ° C während des Desorbtions-und Oxidationsschritts, sowie im Bereich von 100°C bis 500°C während des Sammelschritts.

4. Vorrichtung nach Anspruch 3, **dadurch gekennzeichnet, dass** die Oxidationszone innerhalb des Quarzrohres aus einer Parallel- oder einer anderen Quarzstruktur gebildet ist.

5. Vorrichtung nach Anspruch 3, **dadurch gekennzeichnet, dass** die Oxidationszone ein leeren Teil einer vorgegebenen Länge des Quarzrohres stromab von der Sammelzone ist.

6. Vorrichtung nach Anspruch 3, **dadurch gekennzeichnet, dass** die Oxidationszone mit einer oder mehreren katalytischen Verbindungen wie Basismetalloxide, Titandioxid, Metalle aus Platingruppe oder anderen katalytischen Verbindungen, wobei die Temperatur der Oxidationszone während der Desorption und Oxidation im Bereich von 350 ° C bis 900 ° C liegt.

## Revendications

1. Procédé pour la détection du mercure élémentaire gazeux dans l'air ou dans d'autres gaz, comprenant les étapes suivantes :
- la collection de mercure élémentaire gazeux en faisant passer un volume prédéterminé d'un échantillon d'air ou d'autres gaz à travers une zone de collecte formée à l'intérieur d'un tube en quartz, dans lequel ladite zone de collecte est réalisée sous la forme d'une structure en or.
- le maintien d'une température prédéterminée de la zone de collecte durant l'étape de collecte,
- la désorption du mercure élémentaire gazeux recueilli en chauffant la zone de collection du tube en quartz à la température de désorption,
- la transmission du mercure élémentaire gazeux désorbé par un courant de gaz porteur à un analyseur de mercure,
- le passage du mercure élémentaire gazeux désorbé avec également d'autres mélanges gazeux potentiellement désorbés dans l'air ou d'autres gaz avant de les transmettre à un analyseur de mercure à travers une zone d'oxydation,
**caractérisé en ce que**
la zone d'oxydation est chauffée avant l'étape de désorption et maintenue à une température dans une plage allant de 350°C à 1000°C, préférablement dans une plage allant de 900°C à 1000°C, et plus préférablement à 960°C durant l'étape de désorption et d'oxydation, tandis que la température de la zone d'oxydation est maintenue dans une plage allant de la température ambiante à 500°C durant l'étape de collecte,
et le gaz porteur, transmettant le mercure élémentaire gazeux désorbé avec également d'autres mélanges gazeux potentiellement désorbés à partir de la zone de collecte à travers la zone d'oxydation jusqu'à l'analyseur de mercure,
possède une teneur en oxygène non inférieure à celle contenue dans l'air.

2. Procédé selon la revendication 1, **caractérisé en ce que** la zone de collecte du tube en quartz durant l'étape de collecte est maintenue à une température comprise dans une plage allant de 100°C à 220°C, préférablement à 200°C.

3. Dispositif pour la détection du mercure élémentaire gazeux dans l'air ou dans d'autres gaz, comprenant :
- un tube en quartz (1), à l'intérieur duquel une zone de collection est formée à partir d'une structure en or (2) pour la collection puis par la suite la désorption du mercure élémentaire gazeux d'un échantillon d'air ou d'autres gaz qui passe à travers le tube en quartz (1),
- un moyen de chauffage (3) pour maintenir une température prédéterminée de la zone de collecte durant la collection et pour le chauffage et le maintien de la température prédéterminée pendant la désorption,
- un moyen pour faire passer le mercure élémentaire gazeux désorbé dans l'analyseur de mercure par un courant de gaz porteur,
**caractérisé en ce qu'une** zone d'oxydation est formée en aval de la zone de collecte à l'intérieur du tube en quartz (1), qui est muni d'un dispositif de chauffage séparé (8) pour chauffer et maintenir une température prédéterminée de la zone d'oxydation dans une plage allant de 350°C à 1000°C, préférablement dans une plage allant de 900°C à 1000°C, plus préférablement à 960°C durant l'étape de désorption et d'oxydation, et dans une plage allant de 100°C à 500°C durant l'étape de collecte.

4. Dispositif selon la revendication 3, **caractérisé en ce que** la zone d'oxydation à l'intérieur du tube en quartz est formée à partir d'une structure parallèle ou de toute autre structure en quartz.

5. Dispositif selon la revendication 3, **caractérisé en ce que** la zone d'oxydation est une partie vide de la longueur prédéterminée du tube en quartz en aval de la zone de collecte.

6. Dispositif selon la revendication 3, **caractérisé en ce que** la zone d'oxydation est formée avec un ou plusieurs composés catalytiques tels que les oxydes de métaux de base, le dioxyde de titane, les métaux du groupe du platine ou d'autres composés catalytiques, tandis que la température de la zone d'oxydation durant les étapes de désorption et d'oxydation est maintenue dans une plage allant de 350°C à 900°C.
